Europäisches Patentamt

(19) European Patent Office

Office européen des brevets

(11) Publication number: **0 265 214**
**A2**

## (12) EUROPEAN PATENT APPLICATION

(21) Application number: 87309230.8

(51) Int. Cl.⁴: **A 61 K 37/26**

(22) Date of filing: **19.10.87**

(30) Priority: **20.10.86 DK 5034/86**
**01.12.86 DK 5756/86**

(43) Date of publication of application:
**27.04.88 Bulletin 88/17**

(84) Designated Contracting States:
**AT BE CH DE ES FR GB GR IT LI LU NL SE**

(71) Applicant: **NOVO INDUSTRI A/S**
**Novo Allé**
**DK-2880 Bagsvaerd (DK)**

(72) Inventor: **Skelbaek-Pedersen, Bent**
**13 Skytten**
**DK-3650 Olstykke (DK)**

(74) Representative: **Brown, John David et al**
**FORRESTER & BOEHMERT Widenmayerstrasse 4/l**
**D-8000 München 22 (DE)**

(54) **Polypeptide preparation.**

(57) Protamine zinc insulin preparations containing small crystals of substantially uniform size and no or a minor amount of amorphous material can be prepared by using a lower amount of zinc than used in the known protamine zinc insulin preparations.

EP 0 265 214 A2

## Description

The present invention relates to novel protamine zinc insulin preparations containing small crystals of substantially uniform size and containing no or a minor amount of amorphous material.

In the treatment of diabetes mellitus, many varieties of insulin preparations have been suggested and used. Some of the preparations are fast acting and other preparations have more or less prolonged actions. Preparations with prolonged action have been made in various ways using different components.

An example of insulin preparations with prolonged action is a suspension of zinc insulin crystals. The time of duration for such preparations depends upon the species of insulin, bovine insulin giving the longest time of duration, porcine insulin giving a shorter time of duration and human insulin giving the shortest time of duration. Some people have a need for a human insulin preparation with a more prolonged action than can be obtained with known suspensions of zinc insulin crystals of human species.

Protamine zinc insulin preparations were first described around 1936. During the following decades, protamine zinc insulin preparations with different amounts of protamine, zinc and insulin were prepared. Compared with the protamine zinc insulin preparations of this invention, all the known protamine zinc insulin preparations containing a substantial amount of dissolved protamine contain a substantially higher amount of zinc referring to the content of protamine (vide Tables IV and VIII below).

The known protamine zinc insulin preparations contain crystals of very varying particle sizes (for example crystals of a length between 5 and 50 μm, vide Table VII and VIII below) and, furthermore, they contain a substantial and very varying amount of amorphous material. Often, the ratio between the amount of crystals and of amorphous material in the known preparations will change during storage. Hence, such preparations are physically unstable and this will probably give different absorption rates depending on the time of storage. The market for the known protamine zinc insulin preparations has been decreasing for many years. Furthermore, such preparations have been cancelled from the addendum 1983 to British Pharmacopia 1980.

It is presumed that the particle size is of importance for the absorption of the crystals. It is furthermore presumed that the rate of absorption is more predictable if the crystals have substantially uniform particel size.

In British Patent Specification No. 889,769 a zinc protamine insulin crystal preparation wherein the protamine content of the suspension medium is below 0.0025 mg per ml is described, see Claim 6. This known preparation, which is substantially free of protamine in the suspension medium, deviates from the preparation of this invention, which contains a substantial amount of dissolved protamine. Hence, the two preparations are not of a similar type and function.

One object of this invention is to prepare protamine zinc insulin preparations containing small crystals.

A second object of this invention is to prepare protamine zinc insulin preparations with crystals of substantially uniform particle size.

A third object of this invention is to prepare preparations containing no or only a minor amount of amorphous material.

A fourth object of this invention is to prepare protamine zinc insulin preparations which compared with known human insulin preparations have a very prolonged action.

It has now surprisingly been found that protamine zinc insulin preparations containing small crystals of substantially uniform particle size and containing no or a minor amount of amorphous material can be prepared by using a lower content of zinc than used in the known protamine zinc insulin preparations.

This invention relates to a protamine zinc insulin preparation wherein at least 90% (weight/weight) of the solid material is in crystal form and wherein, preferably, at least 90% (weight/weight) of the crystals have a length below about 20 micrometer (mμ).

According to a preferred embodiment of this invention, the protamine zinc insulin preparations of this invention contain about 0.8 to 1.4 mg of protamine per 100 IU of insulin and an approximate amount of zinc which can be calculated from the following equation:

$$a = 182 \times b - 44 +/- c$$

wherein a is the amount of zinc in μg per 100 IU insulin, b is the amount of protamine in mg per 100 IU insulin, and c is a figure not above 30. Hence, the amount of zinc in mg per 100 IU insulin in the preparations of this invention is between $182 \times b - 14$ and $182 \times b - 74$ (wherein $182 \times b$ is 182 multiplied by the amount of protamine in mg per 100 IU insulin).

According to a further preferred embodiment of this invention, c is not above 20 and this especially applies when the insulin species is porcine or bovine. However, it may also be preferred that c is not above 20 in case the insulin species is human. According to another preferred embodiment of this invention, c is not above 10.

The insulin preparation of this invention has a certain content of protamine in the supernatant. Preferably, the supernatant contains more than 0.005, more preferred more than 0.01, mg protamine per ml and, preferably, the supernatant contains less than 5, more preferred less than 1, mg protamine per ml.

The preparations of this invention wherein the species of insulin is human have a substantially more prolonged action than the known suspensions of zinc insulin crystals of human species.

Protamine is known to be a heterogeneous mixture. Protamine can be obtained from fishes such as Oncorhynchus keta. However, also protamine from other fishes can be used. Normally, protamine is marketed as protamine sulphate. However, also other salts can be used. Preferably, protamine of high purity is used.

The insulin species can for example be bovine, porcine or human, preferably porcine or human, most preferred human. It is also possible to use insulin derivatives which deviates from mammalian insulin in that one or more of the amino acid residues have been exchanged with other amino acid residues. Preferably, insulin of high purity is used. Preferably, the content of insulin in the protamine zinc insulin preparations of this invention is 20 - 500 IU/ml, more preferred 30 - 200 IU/ml, most preferred 40 - 100 IU/ml.

Preferably, the protamine zinc insulin preparations of this invention have a pH value in the range 6.5 - 8, more preferred 7 - 7.5, most preferred 7.2 - 7.4.

According to one preferred embodiment of this invention, the protamine zinc insulin preparations contain no or a minor amount of amorphous material and, preferably, the amount of amorphous material is below about 10%, more preferred below about 5%, most preferred below about 2%, the percentages being based upon the amount of crystals plus amorphous material (w/w).

According to another preferred embodiment of this invention, the protamine zinc insulin preparations contain crystals of substantially uniform particle size. Preferably, most of the crystals have a length below about 20 μm, preferably below about 10 μm. According to a still further preferred embodiment of this invention, more than about 90% of the crystals have a length below about 20 μm, preferably a length below about 10 μm, the percentages being based upon the total content of crystals (w/w).

According to a still further preferred embodiment of this invention, more than 90% of the crystals have a breadth below about 5 μm, preferably a breadth below about 2 μm, the percentages being based upon the total content of crystals (weight/weight).

The insulin preparations of this invention can be prepared by mixing the following constituents: insulin, protamine, a zinc salt, a preservative, an isotonic agent and a buffer. One way of doing this is to prepare an acidic solution (hydrochloric acid) containing insulin, protamine sulphate, zinc chloride, phenol and glycerol. Thereafter, this solution is mixed slowly with a sodium phosphate solution with stirring at room temperature. After mixing, the pH value of the preparation is, if necessary, adjusted to about 7.3. Examples of specific zinc salts are zinc chloride, zinc sulphate and zinc acetate. Examples of preservatives are phenol and m-cresol. An example of an isotonic agent is glycerol. An example of a buffer is sodium phosphate.

The preservative may be phenol or m-cresol. The isotonic agent may be glycerol. The buffer may be disodiummonohydrogenphosphate.

The preparations of this invention are used in the treatment of diabetes. It is recommended that the dosage of the preparations of this invention is selected by a physisian similarly to the selection of the dosage of other insulin preparations.

This invention is further illustrated in the following examples.

Example 1

90 ml of an acidic stock solution designated A was mixed slowly with 10 ml of a stock solution designated B with stirring at room temperature. The amount of constituents in solution A was selected so that the final mixture contained 100 IU insulin per ml, 2.5 mg of phenol per ml, 16 mg of glycerol per ml and the amount of protamine sulphate and zinc stated in Tables I - III below. The zinc salt used was zinc chloride and the protamine sulphate used contained about 19% (w/w) sulphate. Solution B contained 25 mg of disodium hydrogen phosphate ($Na_2HPO_4,2H_2O$) per ml and an amount of sodium hydroxide so that the final mixture had a pH value of about 7.3.

In Tables I - III, the particle size of the protamine zinc insulin crystals is given as the longest and shortest crystals and the broadest and thinnest crystals. Furthermore, the evaluation of the amount of amorphous material in the samples is indicated by a number of stars. One star indicates a trace of amorphous material, two stars indicate a bigger amount of amorphous material etc. No star indicates that no amorphous material was observed.

3

Table I

Human insulin

| Protamine sulphate, mg/ml | Zinc, µg/ml | Particle size, length x breadth, µm x µm | Amount of amorphous material |
|---|---|---|---|
| 1.05 | 95 | 3.4-5.1 x 0.4-0.9 | * |
| " | 125 | 3.4-5.1 x 0.4-0.9 | * |
| 1.25 | 120 | 3.4-5.1 x 0.4-0.9 | |
| " | 140 | 3.4-5.1 x 0.4-0.9 | |
| " | 165 | 3.4-5.1 x 0.4-0.9 | |
| 1.45 | 160 | 3.4-5.1 x 0.4-0.9 | |
| " | 185 | 3.4-5.1 x 0.4-0.9 | |
| 1.65 | 180 | 3.4-5.1 x 0.4-0.9 | |
| " | 195 | 3.4-5.1 x 0.4-0.9 | |
| " | 220 | 3.4-5.1 x 0.4-0.9 | |

Table II

Porcine insulin

| Protamine sulphate, mg/ml | Zinc, µg/ml | Particle size, length x breadth, µm x µm | Amount of amorphous material |
|---|---|---|---|
| 1.25 | 140 | 3.4-7.7 x 0.4-1.2 | * |
| 1.45 | 170 | 3.4-6.8 x 0.4-0.9 | * |
| 1.65 | 200 | 3.4-8.5 x 0.4-0.9 | ** |

Table III

Bovine insulin

| Protamine sulphate, mg/ml | Zinc, µg/ml | Particle size, length x breadth, µm x µm | Amount of amorphous material |
|---|---|---|---|
| 1.25 | 140 | 3.4-10.2 x 0.4-1.7 | ** |
| 1.45 | 170 | 3.4-10.2 x 0.4-1.7 | *** |
| 1.65 | 200 | 5.1-10.2 x 0.9-2.6 | *** |

Example 2

In Table IV, the content of protamine sulphate, of protamine and of zinc, all per 100 IU insulin, is given for some protamine zinc insulin preparations according to this invention.

Table IV

| Protamine sulphate, mg | Protamine, mg | Zinc range, µg | Zinc mean, µg |
|---|---|---|---|
| 0.99 | 0.8 | 82 - 122 | 102 |
| 1.25 | 1.01 | 120 - 160 | 140 |
| 1.45 | 1.175 | 150 - 190 | 170 |
| 1.65 | 1.34 | 180 - 220 | 200 |
| 1.73 | 1.4 | 191 - 231 | 211 |

Example 3

Preparations prepared as described in Example 1 were analysed for content of protamine in the supernatant and the results appear from Table V. The content of nitrogen was measured and it was assumed that the content of human insulin in the supernatant was inferior.

Table V

| Protamine sulphate, mg/ml | Zinc, µg/ml | Free protamine sulphate in the supernatant, mg/ml |
|---|---|---|
| 1.0 | 72 | 0.31 |
| 1.0 | 132 | 0.06 |
| 1.45 | 160 | 0.14 |
| 1.73 | 181 | 0.35 |
| 1.73 | 241 | 0.07 |

Example 4

Ten insulin-dependent type 1 diabetics were used in this study. The study was a random-order open cross-over design with each patient studied twice, interrupted by a period of 10 days. Insulin administered was 125I (mono A14) labelled Ultratard® (human) and a human insulin preparation according to this invention (hereinafter designated Ultraphane®), both 100 IU/ml. For each study, the patients received 24 IU in the right thigh and 6 IU of the same preparation in the left thigh. The results obtained appear from Table VI.

Table VI

| Preparation | Half time, average |
|---|---|
| Ultraphane 6 U | 18 |
| Ultraphane 24 U | 27 |
| Ultratard 6 U | 12 |
| Ultratard 24 U | 13 |

As appears from this table, the preparation of this invention is absorbed significantly slower than the known zinc insulin preparation.

Example 5

Analogously as described in Example 1, similar data for known protamine zinc insulin preparations fulfilling the requirements in the British Pharmacopia 1980 are given in Table VII. These preparations were prepared as 100 IU insulin per ml.

6

Table VII

Bovine/porcine insulin

| Protamine sulphate, mg/ml | Zinc, µg/ml | Particle size, length x breadth, µm x µm | Amount of amorphous material |
|---|---|---|---|
| 1.30 | 230 | 8.5-27.2 x 1.7-3.4 | ***** |
| 1.40 | 230 | 11.9-51.0 x 3.4-5.1 | ***** |

Example 6

Analysis of known protamine zinc insulin preparations appear from Table VIII.

Table VIII

| Preparation | Insulin, IU/ml | Zinc, µg/100 IU | Protamine sulphate, mg/100 IU | Particle size, length x breadth, µm x µm | Amount of amorphous material |
|---|---|---|---|---|---|
| A | 100 | 185 | 1.25 | | |
| B | 40 | 230 | 1.4 | | |
| C | 80 | 230 | 1.3 | | |
| D | 40 | 225 | 1.25 | | |
| E | 100 | 225 | 1.25 | | |
| F | 100 | 225 | 1.65 | 8-35 x 2-5 | ***** |
| G | 100 | 185 | 1.25 | 5-30 x 2-4 | ***** |
| H | 100 | 175 | 1.0 | 5-30 x 2.5-4 | ***** |
| I | 100 | 175 | 1.0 | 5-40 x 2.5-5 | ***** |

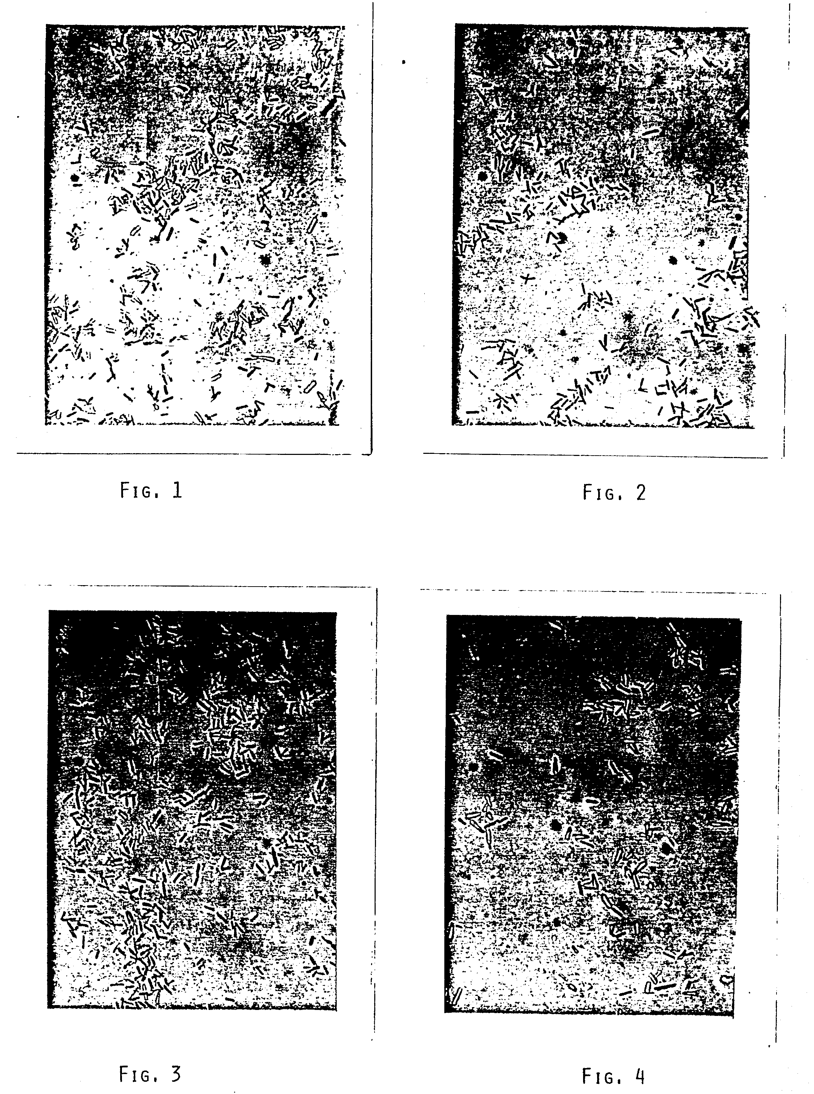

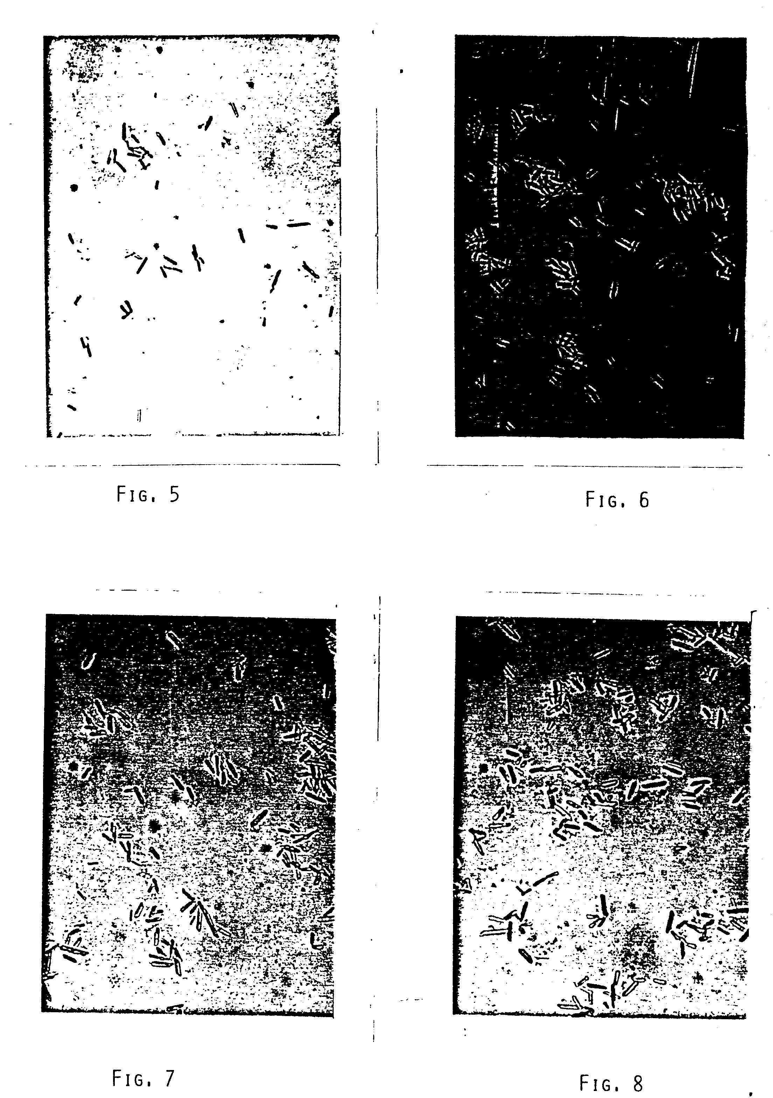

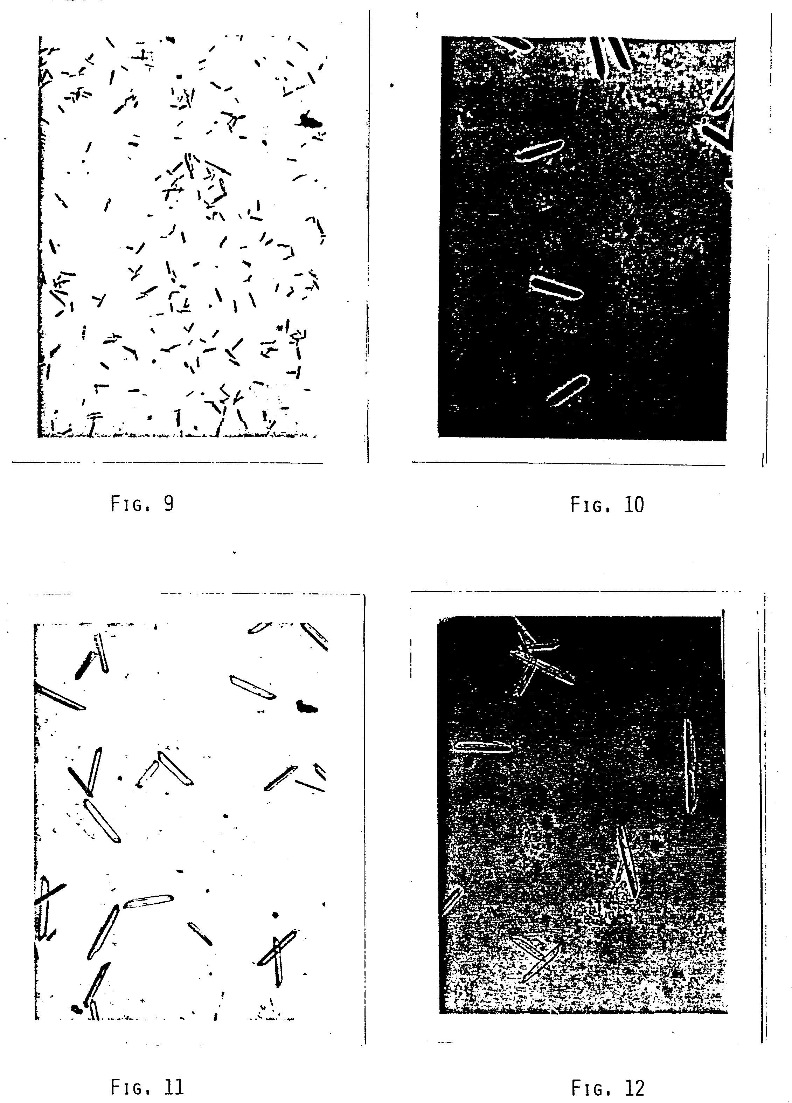

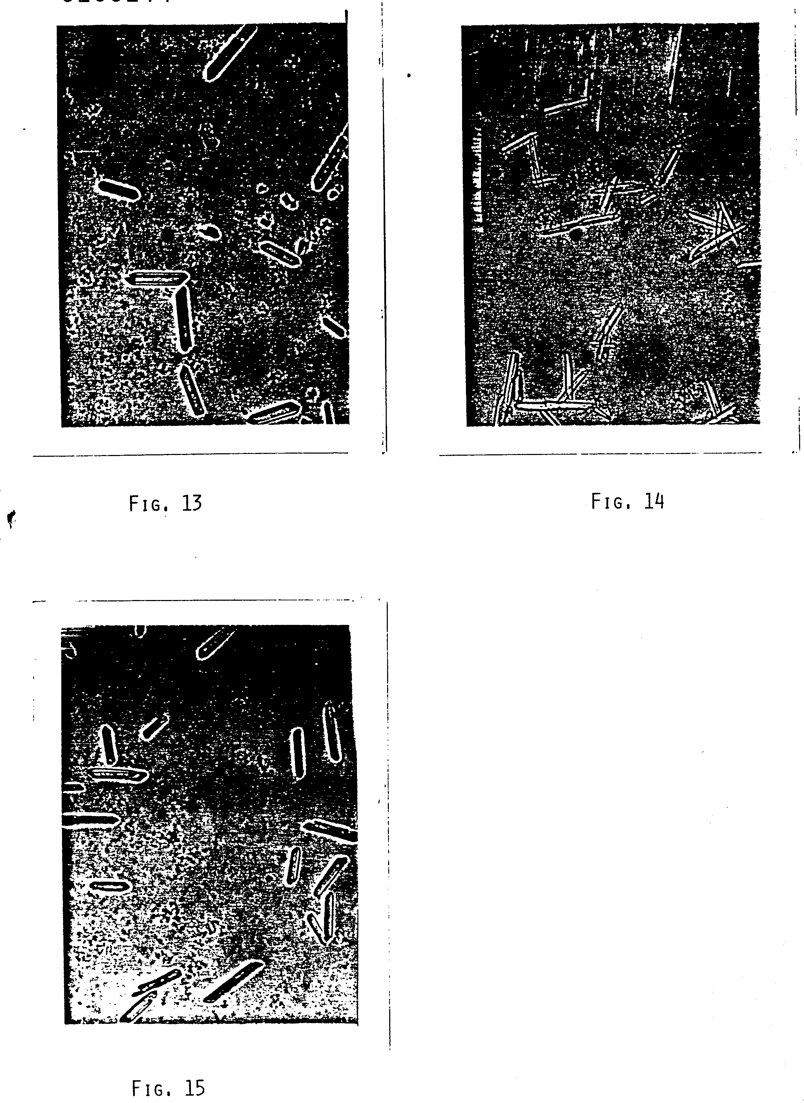

The attached figures (1/4 - 4/4) are photographs of preparations according to this invention (Fig. 1 - 9) and of known preparations (Fig. 10 - 15) enlarged about 400 times. Further data for these preparations appear from Table IX.

Table IX

Data for the preparations on Fig. 1 - 15.

| Fig. No. | Protamine sulphate, mg/ml | Zinc µg/ml | Amount of amorphous material |
|---|---|---|---|
| 1 | 1.45 | 160 | |
| 2 | 1.25 | 140 | |
| 3 | 1.65 | 195 | |
| 4 | 1.25 | 140 | * |
| 5 | 1.45 | 170 | * |
| 6 | 1.65 | 200 | ** |
| 7 | 1.25 | 140 | ** |
| 8 | 1.45 | 170 | *** |
| 9 | 1.65 | 200 | *** |
| 10 | 1.30 | 230 | ***** |
| 11 | 1.40 | 230 | ***** |
| 12 | 1.0 | 175 | ***** |
| 13 | 1.65 | 225 | ***** |
| 14 | 1.0 | 175 | ***** |
| 15 | 1.25 | 185 | ***** |

The features disclosed in the foregoing description, in the following claims and/or in the accompanying figures (photographs) may, both separately and in any combination thereof, be material for realising the invention in diverse forms thereof.

## Claims

1. A protamine zinc insulin preparation wherein at least 90% (weight/weight) of the solid is in crystal form and wherein preferably at least 90% (weight/weight) of the crystals have a length below about 20 micrometers (mµ).

2. Protamine zinc insulin preparation, optionally according to Claim 1, characterized in that it contains about 0.8 to 1.4 mg of protamine per 100 IU of insulin and an approximate amount of zinc which can be calculated from the following equation:

$$a = 182 \times b - 44 +/- c$$

wherein a is the amount of zinc in µg per 100 IU insulin and b is the amount of protamine in mg per 100 IU insulin, and c is a figure not above 30.

3. Preparation according to Claim 1 or 2, characterized in that the content of insulin is in the range from about 20 to 500 IU/ml, preferably is in the range from about 30 to 200 IU/ml, most preferred in the range between about 40 and 100 IU/ml.

4. Preparation according to any one of the preceding claims, characterized in that it has a pH value in the range from about 6.5 to 8, preferably a pH value in the range from about 7 to 7.5, more preferred a pH value in the range from about 7.2 to 7.4.

5. Preparation according to any one of the preceding claims, characterized in that it substantially contains crystals of substantially uniform particle size.

6. Preparation according to any one of the preceding claims, characterized in that it contains no or substantially no amorphous material.

7. Preparation according to Claim 6, characterized in that the amount of amorphous material is below about 10%, preferably below about 5%, more preferred is below about 2%, the percentages being based upon the amount of solid material (weight/weight).

8. Preparation according to any one of the preceding claims, characterized in that most of the crystals have a length below about 20 μm, preferably a length below about 10 μm.

9. Preparation according to Claim 8, characterized in that more than about 90% (weight/weight) of the crystals have a length below about 20 μm, preferably a length below about 10 μm.

10. Preparation according to any of the preceding claims, characterized in that c is not above 20, preferably not above 10.

11 Preparation, according to any one of the preceding claims, wherein the supernatant contains more than 0.005 mg protamine per ml, preferably more than 0.01 mg/ml and preferably contains less than 5 mg protamine per ml, preferably less than 1 mg/ml.

12. The use of insulin for preparing a preparation as defined in any one of the claims 1 through 11.

13. Process for preparing protamine zinc insulin preparations stated in any one of the claims 1 through 11, characterized in mixing protamine, zinc and insulin in pertinent amounts and, if necessary, adjusting the pH valve.

14. The use of the preparation defined in any one of the claims 1 through 11 to diabetics.

0265214

FIG. 1

FIG. 2

FIG. 3

FIG. 4

0265214

FIG. 5

FIG. 6

FIG. 7

FIG. 8

0265214

Fig. 9

Fig. 10

Fig. 11

Fig. 12

0265214

Fig. 13

Fig. 14

Fig. 15